Europäisches Patentamt

European Patent Office

Office européen des brevets

(11) Publication number: **0 227 441**
**A1**

# (12) EUROPEAN PATENT APPLICATION

(21) Application number: 86309907.3

(22) Date of filing: 18.12.86

(51) Int. Cl.⁴: **C 12 Q 1/00**
**C 12 N 1/00, A 61 K 35/66**
**A 61 K 39/00**
**//A61K39/095**

(30) Priority: 20.12.85 US 811784

(43) Date of publication of application:
01.07.87 Bulletin 87/27

(84) Designated Contracting States:
DE FR GB IT NL

(71) Applicant: E.I. DU PONT DE NEMOURS AND COMPANY
1007 Market Street
Wilmington Delaware 19898(US)

(72) Inventor: Hartz, Thomas Peter
957 Rahway Drive
Newark Delaware 19711(US)

(74) Representative: Myerscough, Philip Boyd et al,
J.A. Kemp & Co. 14, South Square Gray's Inn
London, WC1R 5EU(GB)

(54) Preparation of tablets of microbiological substances.

(57) A process for preparing tablets of microbiological substances is provided which results in tablets containing inactivated microorganisms and substantially the same antigenic determinants as did the infectious microorganisms prior to inactivation.

EP 0 227 441 A1

1

## Title
## PREPARATION OF TABLETS OF
## MICROBIOLOGICAL SUBSTANCES

## TECHNICAL FIELD

This invention relates to control products for use in monitoring the performance of assays, especially immunoassays, for the detection of molecular markers indicating the presence of microorganisms in a sample and more particularly to the process for preparing tablets of microbiological substances containing inactivated microorganisms having retained antigenic determinants.

## BACKGROUND ART

Traditionally, culture methods have been employed to detect the presence of microorganisms in samples. A sample is said to be infected with an organism if growth of the microorganism can be demonstrated under the nutrient and environmental conditions necessary to permit or sustain its growth. In addition to culture techniques, several other methods have been described for the detection of infection which do not depend on the growth or microorganisms. These methods generally employ one of several immunoassay techniques, such as latex agglutination, radioimmunoassay, or enzymeimmunoassay to detect the presence of specific antigens derived from the microorganism in a sample. These immunoassay techniques may be applied directly to a specimen taken from a patient or other source, such as food, water, or the atmosphere or they may be applied to a colony isolated from a culture plate which had been inoculated with the sample.

Good laboratory practices dictate that the reagents used in such testing be monitored regularly to assure that the expected results will be obtained both with samples that contain and those that do not contain the organism in question. Microbiology laboratories frequently maintain viable cultures of microorganisms to be used as control materials for such performance testing. This represents a considerable commitment of time, equipment, supplies, and personnel trained in the techniques of microbiology. The maintenance of viable cultures of infective materials poses a health hazard to the laboratorian and, potentially, to the environment. Microbiology laboratories can purchase commercially available control products which contain whole organisms or extracts of organisms. These products are generally available as lyophilized suspensions or solubilized extracts. These control materials typically require reconstitution or dilution steps or other manipulative procedures prior to their use in an assay, and may pose hazards of infectivity to the laboratorian upon reconstitution. The useable lifetime of reconstituted suspensions or solutions of microbial control products is short. Such preparations may settle or aggregate on storage. Degradation products, including proteolytic enzymes, present in reconstituted control materials may alter the antigenic properties of the product thereby diminishing the immunoreactivity of the control product in a time-dependent fashion.

Methods of preparing dried materials containing clinical reagents are described in U.S. Patent 3,721,725, issued March 20, 1975, U.S. Patent 3,928,566, issued December 23, 1975, and U.S. Patent 3,932,943, issued January 20, 1976, all to A. R. Briggs et al., and in U.S. Patent 4,211,015, issued July 8, 1980, to T. H. Adams, et al. The Briggs et al. patents disclose the use of a freeze-spraying process followed by lyophilization to prepare powder blends containing biological materials dispersed uniformly throughout the blend. These materials include pharmaceuticals, proteins, enzymes, gelatin, serum and plasma. U.S. Patent 3,721,725 suggests that tablets can be formed from these dried blends. U.S. Patent 4,211,015 describes a method and apparatus for the preparation of frozen particulates from liquid feedstock to produce lyophilized blends containing uniform distribution of biologically active substances such as pharmaceuticals, proteins including antigens, antibodies and enzymes, organisms including bacteria and viruses, and body fluids. The described process is not amenable to retaining the antigenic determinants of inactivated organisms.

Berman et al. described the preparation of tablets containing antigens and antisera for use in diagnostic laboratories [Berman et al., J. Clin. Microbiol., Volume 5(4), 492-493 (1977)]. Tablets were formed from freeze-dried concentrated antigen or antiserum preparations with NaCl added to provide adequate bulk. Each of the antigen tablets prepared contained a quantity of material sufficient to prepare 20 mL of antigen preparation upon rehydration. This process is not suitable for production of single dose tablets.

There are no currently known procedures for the preparation of inactivated microorganisms which either during preparation or upon reconstitution retain those antigenic determinants, present in or on the viable microorganism, necessary for immunorecognition.

Microbiological assays are generally designed to yield positive results with a minimum of antigen. Immunoassay detection sensitivity as low as $10^{-13}$ M/L is not uncommon. This requires that minute quantities of antigen be present in each single dose tablet. The use of these materials as control products requires that these minute quantities of antigen be present in each tablet in identical quantities. There is demonstrated need for the preparation of small quantities of antigen, representing a single assay equivalent, in a tablet form with the reproducibility required of a control product.

It is the purpose of this invention to prepare a non-infective tabletted control product for use in monitoring the performance of assays used to detect the presence of microorganisms in a sample. The quantity of biological material in each tablet should be adequate for a single determination and, preferably, the tablets should be applicable directly in the assay with no need for predissolution or dilution.

## DISCLOSURE OF THE INVENTION

A process for preparing tablets of microbiological substances containing inactivated microorganisms and substantially the same antigenic determinants as the infectious microorganism comprising the steps of:

    a.  preparing a suspension of said microorganism and determining its concentration;

    b.  inactivating the microorganism;

    c.  preparing a dried blend of the inactivated microorganism with excipients; and

    d.  tabletting the blend prepared in step (c) to contain the desired amount of antigenic determinants.

5

## DESCRIPTION OF THE INVENTION

Organisms can be grown using routine microbiological techniques under nutrient and environmental conditions necessary to support their growth. After an adequate number of microorganisms, such as bacteria, viruses, chlamydia, mycoplasma, protozoa and rickettsia, are present, they are suspended in an aqueous buffer solution. The concentration of microorganisms in the suspension can be estimated spectrophotometrically and adjusted to the target concentration as needed. An aliquot of this suspension is held for subsequent quantification of the actual concentration of organisms.

The remainder of the organism suspension can be inactivated by any of several techniques known to destroy viability and result in inactivated microorganisms. By inactivated microorganisms is meant the product resulting from the inactivation process. It is the product of the disruption of the microorganism and includes the antigenic determinants originally present in or on the microorganism and enzymes such as proteases, nucleases, lipases. Such techniques include exposure to heat, ultraviolet radiation (either alone or in conjunction with other agents such as psoralen), specific antimicrobial agents, gases such as oxygen for microorganisms sensitive to oxygen, etc.

After inactivation of the suspension, an aliquot is placed under conditions necessary to support growth, preferably on culture plates, to ascertain that the inactivation process was effective. In parallel, an aliquot of the original suspension collected prior to inactivation is subjected to the same growth conditions to monitor those conditions as well as to provide a means to quantify the number of viable organisms in the original suspension.

6

A dried blend containing tablet excipients and inactivated microorganisms can be prepared using standard physical pharmacy techniques. Excipients are chosen from materials which are soluble in aqueous solution and do not interfere with the assay for which the tablets will be used. Preferred excipients are chosen from the saccharides and include sorbitol, mannitol, maltose, lactose, dextran, inositol, glucose and sucrose. Preferred techniques for formulating the dried blend include fluid bed spray granulation in which a suspension of microorganisms is sprayed over a bed of solid excipient fluidized by the passage of high velocity air or inert gas through the excipients and freeze spraying in which a solution of excipient and microorganisms is sprayed into a container with a moving bath of low boiling liquid such as fluorocarbon refrigerant and lyophilizing the resultant frozen droplets. The resultant blends can be sized to obtain particle sizes appropriate for tabletting by passage through, for example, a 20-30 mesh screen. The addition of, for example, polyethylene glycol at a preferred concentration of approximately 5% by weight by V-blending facilitates the subsequent tabletting operation by acting as a lubricant.

Tablets of the desired size, weight, and hardness can be formed from the dried blend using a standard tablet press such as a Stokes rotary press. Tablet size and weight are chosen for the particular requirements of the assay. Tablet hardness usually represents a compromise between fragility and dissolution rate; a range of 1000±300 grams as measured on a Key International hardness tester HT-300 is preferred. Tablets usually are stored protected from moisture.

The amount of inactivated product required to yield the desired response in an immunoassay can be determined in two ways. In method 1, a series of dilutions of the suspension of inactivated microorganisms is tested in the immunoassay and compared to standard suspensions of infectious organisms. In method 2, a series of tablets is prepared containing varying amounts of inactivated product. These tablets are then tested in the immunoassay and the results compared to results obtained with suspensions of infectious organisms. The amount required will be dependent upon the assay sensitivity and the requirements of clinical utility.

All previously utilized processes involved the introduction of potentially infective materials into the blending apparatus and the degradation during processing of antigenic determinants (molecular markers) present in or on the infectious agent (microorganism) prior to inactivation. Such degradation would result from the "self-digestion" of the antigenic determinants by the enzymes which are part of the homogeneous environment created by the inactivation process which disrupts the microorganisms. The advantage realized by the process of this invention is that the inactivated microorganisms introduced to the blending process are noninfectious. The unexpected advantage is that the inactivated microorganisms retain their biological activity (antigenicity) with respect to assay determination after the blending and tabletting operations. Shearing forces created during spraying processes, proteolytic activity present in the liquid suspension and the exposure of the suspension to ambient or elevated temperatures during fluid bed spray granulation process, surprisingly, do not result in the loss of antigenic activity. While using a technology such as described by Berman et al., supra, in which the antigen preparation is frozen as a bulk liquid and lyophilized, also diminishes loss of antigenicity, it has the inherent disadvantage of not being suitable for the preparation of tablets yielding a single assay equivalent of antigen upon reconstitution of the freeze-dried material.

Example

A clinical isolate of Neisseria gonorrhoeae was grown on chocolate agar plates. A microbiological loop was used to transfer colonies to phosphate buffered saline (PBS), pH 7.4. Colonies were added to the PBS until an optical density of 0.15 at 650nm was attained. An aliquot of the suspension was held for subsequent plating and the remainder of the suspension was placed in a boiling water bath for five minutes. The aliquot of suspension removed prior to heating was diluted 1:1000 and streaked on chocolate agar plates. A total of 20 plates were inoculated with 0.1 mL each of heated suspension (undiluted). The remainder of the heated suspension was stored at -70°C. All plates were incubated at 37°C under 5-10% $CO_2$ for 48 hours and inspected for growth of N. gonorrhoeae. Based on the number of colonies counted on plates containing the unheated suspension, the original suspension contained 2.43 x $10^8$ colony forming units (CFU)/mL. No growth of N. gonorrhoeae was detected on any of the 20 plates inoculated with the heated (inactivated) suspension. Enzyme immunoassays were performed on a series of dilutions of heat-inactivated N. gonorrhoeae. In these assays, samples were incubated with a solution of a conjugate of monoclonal antibody specific for N. gonorrhoeae and horseradish peroxidase and a solid support coated with the same monoclonal antibody. After washing the immune-complex sandwich formed on the solid support, it was incubated with hydrogen peroxide and tetramethylbenzidine. The development of a blue color indicated the presence of N. gonorrhoeae in the sample. The intensity of color formation, which can be quantified by its absorbance at 658nm, is related to the concentration of organism in the sample. The results of the assay on the above series of dilutions of inactivated N. gonorrhoeae indicated that inactivated product resulting from $1 \times 10^6$ organisms per tablet would yield the desired absorbance.

Using the relationship:

$$V = \frac{M \times n}{m \times C}$$

where

V = volume of suspension required (mL)

M = weight of blend to be prepared (grams)

n = number of organisms required for inactivation, per tablet (CFU)

m = weight of each tablet to be prepared (grams)

C = concentration of organisms in suspension prior to inactivation (CFU/mL).

it was determined that to prepare 500g of dried blend to be formed into 0.080g tablets containing the inactivated product resulting from $1 \times 10^6$ organisms each, from a suspension containing $2.43 \times 10^8$ CFU/mL prior to inactivation, 25.7 mL of the suspension would be required.

The frozen suspension of heat-inactivated N. gonorrhoeae was thawed and a 25.7-mL aliquot was diluted to 125 mL with purified water. The diluted suspension was sprayed at 3 mL/minute over a fluidized bed of 475g sorbitol in a fluid bed spray granulation apparatus (Aeromatics, 1 kg unit) at an atomization rate of three bars. An additional 25 mL of purified water was sprayed to clear the lines. The blend was dried for 40 minutes, while fluidizing. Product temperature was maintained at 30-35°C throughout the spraying and drying operation. A total of 469.6g product was collected and sized through a No. 30 mesh screen.

Polyethylene glycol (Carbowax 6000, 24.72g, 5% w/w) was added to the product and mixed by V-blending for 8-10 minutes.

Tablets were formed on a Stokes tablet press at targets of 0.080±2g, width 0.219 inches, height 0.096-0.156 inches, hardness 1000±200g (Key International HT-300). Actual values were 0.080±0.38g, 0.136±0.001 inch thickness, 1072±117g hardness. Tablets were stored in a desiccator at 2-8°C.

The performance of the tablets prepared and background due to tablet excipients were determined using the enzyme immunoassay described above. Results are presented in Table 1 below. In an assay carried out in absence of N. gonorrhoeae, tablets containing 80 mg of sorbitol and 4 mg of Carbowax 6000 were tested. The absorbance at 658 nm was found to be 0.043, a level considered not to be an interferant. The data presented in Table 2 below, when compared to the results shown in Table 1 for the tablets of this invention, show that these tablets do contain the desired and preselected amount of antigenic determinants. (The data for Table 2, average of three determinations, were obtained with suspensions of viable, infectious N. gonorrhoeae.)

## TABLE 1

### RESULTS OF ENZYME IMMUNOASSAYS WITH TABLETS

| SAMPLE | ABSORBANCE AT 658 nm |
|--------|----------------------|
| Tablet #1 | 0.3035 |
| Tablet #2 | 0.3026 |
| Tablet #3 | 0.2945 |
| Tablet #4 | 0.3314 |
| Tablet #5 | 0.3853 |
| Mean ± SD | 0.323 ± 0.037 |

## TABLE 2

### RESULTS OF ENZYME IMMUNOASSAYS WITH INFECTIOUS SUSPENSIONS

| No. of Organisms | Absorbance at 658nm (± SD) |
|------------------|----------------------------|
| $2.98 \times 10^6$ | 1.020 (± 0.104) |
| $2.98 \times 10^5$ | 0.267 (± 0.012) |
| $2.98 \times 10^4$ | 0.067 (± 0.022) |

12

CLAIMS

1.      A process for preparing tablets of microbiological substances containing inactivated microorganisms and substantially the same antigenic determinants as the infectious microorganism before inactivation, which process comprises

        (a)   preparing a suspension of said microorganism and determining its concentration;

        (b)   inactivating the microorganism;

        (c)   preparing a dried blend of the inactivated microorganism with excipients; and

        (d)   tabletting the blend prepared in (c) to contain the desired amount of antigenic determinants.

2.      A process according to claim 2 wherein the microorganism is selected from bacteria, viruses, chlamydia, mycoplasma, protozoa and rickettsia.

3.      A process according to claim 1 or 2 wherein the excipient is a saccharide.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 385 402  (A. MAYR et al.) <br> * Page 2, line 21 - page 3, line 16; page 4, lines 8-13; claims * | 1,2 | C 12 Q   1/00 <br> C 12 N   1/00 <br> A 61 K  35/66 <br> A 61 K  39/00 // <br> A 61 K  39/095 |
| Y | | 1-3 | |
| | --- | | |
| Y | GB-A-  806 949  (I.C.I. LTD) <br> * Page 1, line 1 - page  2,  line 119; claims * | 1-3 | |
| | --- | | |
| X | FR-A-2 494 963  (FROMAGERIES BEL) <br> * Page 2, line 18 - page 6, line 30; claims 1-4 * | 1 | |
| | --- | | TECHNICAL FIELDS SEARCHED (Int. Cl.4) |
| Y | EP-A-0 086 071  (THE REGENTS OF THE UNIVERSITY OF CALIFORNIA) <br> * Abstract; page 1, lines  22-31; page  2,  line  11 - page 4, line 13; page 5, line  15  -  page  6, line 12; claims * | 1-3 | C 12 Q <br> C 12 N <br> C 12 C <br> A 61 K <br> G 01 N |
| | --- | | |
| Y | FR-A-2 104 349  (E.I. DU PONT DE NEMOURS & CO.) <br> * Page  1, line 1 - page 3, line 10 * | 1-3 | |
| | ---       -/- | | |

The present search report has been drawn up for all claims

| Place of search | Date of completion of the search | Examiner |
|---|---|---|
| THE HAGUE | 18-03-1987 | HITCHEN C.E. |

European Patent
Office

EUROPEAN SEARCH REPORT

**0227441**
Application number

EP  86 30 9907

## DOCUMENTS CONSIDERED TO BE RELEVANT

Page  2

| Category | Citation of document with indication, where appropriate, of relevant passages | Relevant to claim | CLASSIFICATION OF THE APPLICATION (Int. Cl.4) |
|---|---|---|---|
| A | GB-A-1 108 524  (GENERAL ELECTRIC CO.) <br> * Page 1, line 11 - page 2,  line 71; page 3, lines 10-16 * | 1,2 | |

-----

TECHNICAL FIELDS SEARCHED (Int. Cl.4)

The present search report has been drawn up for all claims

| Place of search | Date of completion of the search | Examiner |
|---|---|---|
| THE HAGUE | 18-03-1987 | HITCHEN C.E. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

Form 1503 03 82